# EUROPEAN PATENT APPLICATION

(11) **EP 3 882 637 A1**
(43) Date of publication of application: **22.09.2021**
(21) Application number: 19883439.2
(22) Date of filing: 03.04.2019
(51) Int. Cl.: G01N 35/08, G01N 33/53, G01N 1/38

(54) **MICROFLUIDIC SYSTEM SUITABLE FOR LIQUID MIXING, AND METHOD**

(30) Priority: 16.11.2018 CN 201811365513
(71) Applicant: Nanjing Aisi Weizhi Biotechnology Co., Ltd., Nanjing, Jiangsu 210038 (CN)
(72) Inventor: JENKINS, Gareth, Hertfordshire CM233NG (GB); YANG, Xin, Nanjing, Jiangsu 211100 (CN)
(74) Representative: Schlich, George
(86) International application number: PCT/CN2019/081187
(87) International publication number: WO 2020/098206

(57) **Abstract**

A microfluidic system and method suitable for liquid mixing. The microfluidic system uses a pump (400) as the driving source, which draws at least two liquid samples that are to be mixed into the pump (400). Some air is drawn into the pump (400) as well. The system is also comprised of a mixing reservoir (203). The two liquids drawn into the pump (400) are pushed into the mixing reservoir (203). The air bubbles generated by the air have a stirring effect on the mixed liquid in the mixing reservoir (203). After the air bubbles burst, left at rest, and the air has risen to the top of the mixing reservoir (203), the mixed liquid is drawn back to the pump (400) and fed to the outlet (103) for subsequent detection steps. The addition of an antifoaming agent will prevent the accumulation of air bubbles during the mixing process. In the system, the valves (501, 502, 503, 504) and the sensors (601, 602, 603, 604) in the microfluidic channels (301, 302, 303, 304) will be used for the operation of the microfluidic system and for the precise control of the flow.

## Description

### Technical Field

The present invention relates to a microfluidic system, in particular to a microfluidic system suitable for liquid mixing and a liquid mixing method.

### Background Technology

A microfluidic system with chips or components can automate the time-consuming and error-causing sample processing. Pumps and valves can be used in a typical system with chips and components to drive the liquid flow, so as to obtain reliable and controllable preparation of samples and facilitate the subsequent detection steps. The mixing preparation of different samples or the mixing of the sample and a buffer solution is a commonly used step in some microfluidic systems, but the main challenge of the current microfluidic systems is that it is difficult to effectively mix different liquids on integrated microfluidic chips. Due to the typical low Reynolds number in microfluidic systems, it is usually difficult to achieve turbulent mixing. General microfluidic methods use T-shaped or Y-shaped channel diffusion to mix the fluid, but diffusion mixing takes a long time and has poor mixing efficiency. In order to enhance the diffusion effect, the common approach is to increase the convex structure in the channel or to use other structures that increase the local Reynolds number of the fluid, so as to achieve the effect of turbulence and enhance the mixing ability. However, this method results in increased structure complexity, reduced reliability, as well as increased manufacturing cost and processing difficulty of the microfluidic chips.

On the other hand, features such as the high surface tension of air bubbles that are mixed or generated in the channels, small channel size, etc. in integrated microfluidic systems hinder the liquid flow, which affect the liquid flow efficiency and may have unpredictable effects on the subsequent detection, which is not desirable for the designer.

### Summary of the Invention

In order to solve the aforementioned problems, the present invention provides a microfluidic system and a method for mixing liquid using the described microfluidic system.

The microfluidic system of the present invention includes multiple fluid inlets, one or more mixing reservoirs, a corresponding number of fluid sensors, one or more pumps to provide liquid driving force and multiple valves to control the microfluidic liquid flow paths.

According to one aspect of the present invention, the microfluidic system of the present invention is comprised of at least two fluid inlets which are connected to the microfluidic channels.

At least one pump is connected to the described microfluidic channel to apply positive pressure or negative pressure to the microfluidic channel so that the sample or liquid can be transferred through the described microfluidic channel, and at least one described pump is also connected to the fluid outlet through the microfluidic channel.

At least one valve is set up in one or more of the described microfluidic channels, preferably near the fluid inlet or outlet, to selectively control the opening and closing of the microfluidic channel.

At least one mixing reservoir is connected to the described pump through the microfluidic channel.

Preferably, at least one liquid level sensor is also included, at least one described liquid level sensor detects the fluid flow in order to control the action of the pump and valves when the volume of the described liquid is drawn in by the pump.

According to another aspect of the present invention, at least the first sample container and the second sample container are included. At least the first sample container and the second sample container are used to hold different liquids. The liquid in the sample containers is connected to the microfluidic channels through the described fluid inlet, and the liquid in the sample containers can be transferred through the described microfluidic channels using the action of the described pump.

According to the second aspect of the present invention, a method for mixing liquid in a microfluidic system is provided. The driving source in the microfluidic system pushes the liquid samples to be mixed into the mixing reservoir from the respective fluid inlets through the microfluidic channels.

The liquid drawn in by at least one fluid inlet is mixed with a certain amount of air; when the driving source pushes the liquid into the mixing reservoir, the air generates air bubbles, flows through the mixed liquid and rises to the top of the mixing reservoir.

The driving source draws out the mixed liquid from the mixing reservoir and transfers it to the next channel.

Preferably, a pump is used as the described driving source.

Preferably, a liquid level sensor is set up in the mixing reservoir. When the mixed liquid is drawn out from the mixing reservoir, the valve connected to the mixing reservoir and the pump is closed before the air is drawn out.

Preferably, the described driving source is connected to the outlet channel, in which a sensor is set up to calculate and control the volume of the mixed liquid after being drawn out.

Preferably, an antifoaming agent is added so that no excess air bubbles in the mixed liquid accumulate at the top of the mixing reservoir; the antifoaming agent can be pre-added to the mixing reservoir, pump or liquid sample to be mixed.

The microfluidic system of the present invention provides an effective and cost-saving system and a method for the mixing of the sample and buffer solution or the mixing of different samples. Without the aid of extra structure in the microfluidic channel, when the air drawn into the device is being pushed by the driving source to the mixing reservoir through the microfluidic channel, air bubbles will form and penetrate the mixed liquid and stir the mixed liquid evenly. At the same time, the presence of the mixing reservoir makes the excess air in the mixed liquid rises to the liquid surface, and the antifoaming agent can be used to further prevent the accumulation of air bubbles in the device.

Furthermore, when the device of the present invention is applied to sample detection, the buffer solution can be added in the buffer chamber in advance through the built-in automatically controlled pneumatic valve, and the detection can be directly carried out after the sample is added, which greatly simplifies the detection process and improves the detection efficiency.

### Description of the Drawings

Figure 1 is a typical structure of the described microfluidic system in the present invention.
Figure 2 is another embodiment of the microfluidic system.

### Embodiment

One or more specific embodiments of the present invention are described below. It should be appreciated that in any engineering or design project, any such improvement in the actual implementation plan and numerous implementation measures should achieve the specific objectives of the improver. When the elements in various embodiments of the present invention are presented, the articles 'a', 'an', 'the' and 'described' are intended to indicate the presence of one or more elements. The terms 'include', 'comprised of' and 'have' are intended to be inclusive and indicate that there may be elements other than those listed.

The microfluidic system of the embodiment of the present invention includes at least two fluid inlets, one or more mixing reservoirs, a corresponding number of fluid sensors, one or more pumps to provide liquid driving force and multiple valves to control the microfluidic liquid flow path.

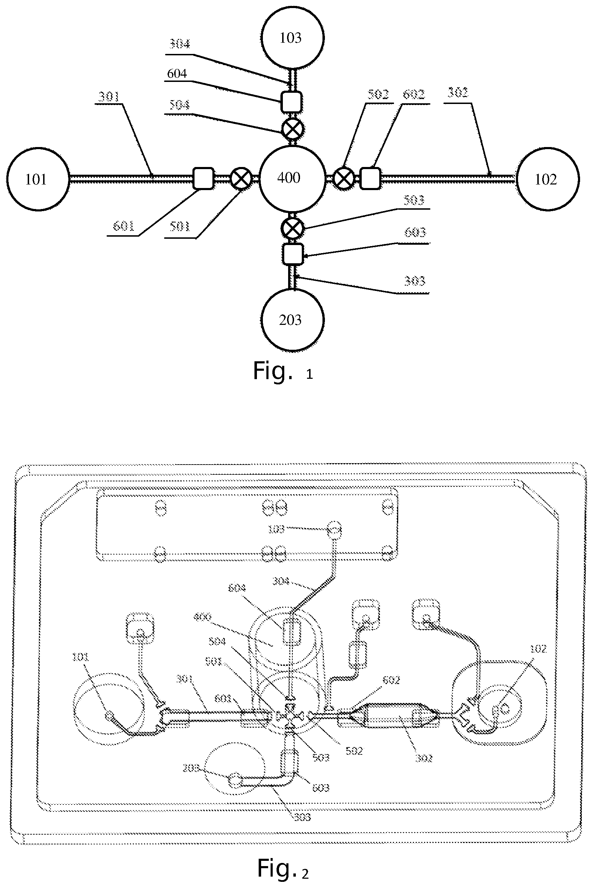

Figure 1 shows the basic and typical structure of the present invention:
The microfluidic system is comprised of microfluidic channels and storage structures for fluid flow operation. This structure is usually achieved by injection moulding, computer numerical control machining, laser processing, cutting, photolithography, 3D printing and other common technologies. The structure can be made of many materials, including but not limited to, thermoplastics, glass, silicon, elastic polymers, etc.

Fluid inlet 101 and fluid inlet 102 are included to respectively connect to sample containers 201 and 202. Fluid inlet 101 and 102 are respectively connected to one end of microfluidic channels 301 and 302; different sample containers 201 and 202 can hold different liquids. The described liquids can include reagents, buffers, samples, etc.; the described sample containers 201 and 202 can be reservoirs, liquid sacs or other types of containers. Depending on the fluid processing function required, such as when more than two liquids need to be mixed, or when processing liquid in other states, any number of inlets and outlets can be added to the microfluidic system to form a more complex system.

Pump 400 is connected to the other end of microfluidic channels 301 and 302, and pump 400 is connected to one end of microfluidic channels 303 and 304, in which the other end of microfluidic channel 304 is connected to fluid outlet 103. Pump 400 applies positive or negative pressure to the microfluidic channel terminal to draw the sample to be mixed into the pump pipe, or to pump the liquid in the pump pipe into the desired microfluidic channel.

There are many types of pumps to choose from, such as an external syringe pump, peristaltic pump, differential pressure air pump, piston pump, electrophoresis pump, etc. An integrated syringe pump is used in one preferred embodiment. The integrated syringe pump consists of a plastic tube connected to the microfluidic channel and a plunger that can pump fluid into or out of the syringe tube. The advantage of using an integrated syringe pump is that the extraction device is mechanically controlled to ensure a stable extraction process.

This embodiment used a single pump to draw the sample, but if necessary, multiple pumps can be used in a more complex structure.

Multiple microfluidic valves, 501, 502, 503, 504, which are respectively set up in microfluidic channels 301, 302, 303, 304 are also included. Pump 400 interacts with valves 501, 502, 503, 504, so that the sample or liquid can be transferred through the desired microfluidic channels, 301, 302, 303, 304; the microfluidic valves are set up near the fluid inlet or outlet to selectively control the opening and closing of the microfluidic channels in a preferred embodiment.

The microfluidic valves can be any type of valves, such as mechanical valves, hydrophobic valves, etc. Pneumatically actuated membrane valves can be used in a preferred embodiment. When the pressure is applied to the thin elastic membrane valve, the valve will press down and block the flow path, thus preventing the liquid flow in the microfluidic channel. The valve is controlled by a vacuum to allow the liquid to flow. The pneumatic valve with built-in automatic control can make the microfluidic system automatically carry out the detection according to the pre-set program after the sample is added, which effectively simplifies the detection process and improves the detection efficiency.

The other end of microfluidic channel 303 is connected to mixing reservoir 203. Mixing reservoir 203 preferably has a cavity structure with a certain height, so as to have a certain space for containing the air at the top. Pump 400 pumps the liquid to be mixed into mixing reservoir 203 through the pump pipe or draws the liquid out from mixing reservoir 203 through the outlet.

In another embodiment, the upper part of mixing reservoir 203 has a channel for discharging the air or the upper part of mixing reservoir 203 is a cavity structure that can be opened.

Liquid level sensor 603 is set up in microfluidic channel 303 to detect the fluid flow in microfluidic channel 303, and its monitoring data are fed back to the control system of the microfluidic system to control the action of pump 400 and the valves to ensure that the desired volume of liquid is fed into mixing reservoir 203.

Similarly, liquid level sensors 601, 602 can also be set up in microfluidic channels 301, 302 to monitor and control the samples to be mixed. Sensor 604 can be set up in microfluidic channel 304 to control the volume of the mixed liquid drawn out from mixing reservoir 203 and to detect whether there are excess air bubbles in the microfluidic channel being drawn to fluid outlet 103.

The aforementioned liquid level sensors 601, 602, 603, 604 can be placed in microfluidic channels 301, 302, 303, 304 to sense whether there is fluid flow in the microfluidic channels. In one embodiment, the infrared proximity sensor below the microfluidic channel is used as the liquid level sensor. In other embodiments, other types of sensors can be selected to be placed below or above the channel or to be integrated into the microfluidic channel.

In another embodiment, a camera imaging system is used instead of a liquid level sensor to detect and monitor the fluid in the entire microfluidic system.

The data monitored by the aforementioned sensors are fed to a control system, such as a microcontroller or a computer. Through the controller's feedback to the monitored data and the control of the predetermined program, the system outputs a control signal to control the corresponding pump and valve, thereby controlling the operation of the microfluidic system.

In different embodiments, a specific quantity of outlets, inlets, pipelines, pipes and terminals, the connection manner, as well as the specific setup of sensors and valves can be changed based on the application.

The use of the aforementioned typical microfluidic system is described in the following embodiments:
During the initial state, the valves of each microfluidic channel are closed. The fluid to be mixed, for example, is stored in sample containers 201, 202 in advance, enters the corresponding microfluidic channel through fluid inlet 101 and fluid inlet 102 respectively; in one embodiment, the sample is in one sample container 201, and the buffer solution is in another sample container 202.

Open valve 501 in the microfluidic channel that connects the pump to fluid inlet 101;
Pump 400 draws the liquid from fluid inlet 101, and the liquid enters the pump pipe through microfluidic channel 301. During the process, liquid level sensor 601 detects the liquid flow and controls the opening and closing of the pump and valve 501 when the required volume is being drawn in.

Close valve 501 and open valve 502 in the microfluidic channel that connects the pump to fluid inlet 102.

The pump 400 draws the liquid from the fluid inlet 102, and the liquid, together with the air, enters the pump pipe through microfluidic channel 302. During the process, liquid level sensor 602 detects the liquid flow and controls the opening and closing of the pump and valve 502 when the required volume is being drawn in.

Close valve 502, and open valve 503 in the microfluidic channel that connects the pump to mixing reservoir 203.

Pump 400 pushes the liquids to be mixed in the pump pipe into mixing reservoir 203. During the process, liquid level sensor 603 detects the liquid flow and controls the opening and closing of the pump and valve 503 when the required volume is being drawn in.

In one embodiment, pump 400 pushes the remaining, or additional air drawn in, into mixing reservoir 203.

When the air drawn in by the described pump 400 is subsequently pushed into the liquid to be mixed, the air generates air bubbles, penetrates the liquid and rises to the top of mixing reservoir 203. During this process, in addition to diffusion, the liquid to be mixed will be additionally stirred when the air bubbles penetrate through, thereby contributing to thorough mixing.

When liquid level sensor 603 detects enough of the air is pumped into mixing reservoir 203, pump 400 will draw out the liquid from mixing reservoir 203 back to the pump pipe. At this point, level sensor 603 detects the liquid flow in the microfluidic channel, so as to allow the desired volume of fluid to be drawn into the pump pipe, while ensuring that valve 503 is closed before the air is drawn out.

In another embodiment, before the liquid in mixing reservoir 203 is drawn back to the pump pipe by pump 400, the air in mixing reservoir 203 is completely discharged through the upper channel or the opening structure of the cavity.

Close valve 503 in the microfluidic channel that connects the mixing reservoir 203 to pump 400, and open valve 504 that connects pump 400 to outlet 103.

Providing power to pump 400 will push the mixed liquid in the pump pipe to the outlet for the next step of analysis. During this process, liquid sensor 604 detects the liquid flow, verifies that there are no air bubbles in the liquid and determines the total volume of the liquid being pushed to the outlet.

In one embodiment, pump 400 or another driving source is used to repeatedly mix the liquid with air bubbles in pump 400 and mixing reservoir 203, thereby using the air bubbles therein to thoroughly mix the liquid that are to be mixed.

In another embodiment, an antifoaming agent is used, which is pre-added to mixing reservoir 203, pump 400 or the fluid to be mixed; when entering mixing reservoir 203, the antifoaming agent can make the air bubbles burst after passing through the liquid, so that they do not accumulate at the top of the mixing pool. In this way, air bubbles that have accumulated in the liquid will be removed before mixing because they will float to the mixing pool surface and burst, and the air will stay at the top of mixing reservoir 203.

In one embodiment, 0.10.1-0.5 microliters of an antifoaming agent is applied to the bottom of pump 400 and the outlet of mixing reservoir 203 respectively to remove the air bubbles generated during the fluid flow.

Antifoaming agents can be used to prevent the generation of excessive air bubbles during the detection. Antifoaming agents are commonly used in industrial and chemical processing applications. For example, it is usually necessary to add surfactants to the liquid being processed because the liquid can easily generate air bubbles as it passes through the microfluidic system. When adding a small amount of antifoaming agent to the device, it can be mixed into the liquid before being added to the device; also, it can be added to mixing reservoir 203 and pump 400. These additions can effectively prevent the formation of air bubbles during the process. In one embodiment, a silicon-based antifoaming agent can be used, but other types of antifoaming agents may also be used depending on the type of fluid.

The aforementioned process describes key aspects of the present invention, including fluid handling, mixing, sensing and the removal of bubbles. By combining the key elements of the system in different ways, a more complex processing method can be achieved, thus realising a more complex flow control system that includes multiple pumps 400 and system elements such as the reservoir, inlet, outlet, sensor, valve, etc.

The following embodiment uses the aforementioned microfluidic system to realise a device for performing automated immunisation. The device has the same basic elements as the example described above, but several additional valves and sensors are added to enable the automatic measurement of samples, as shown in Figure 2.

One of the liquid inlets, a, draws in the liquid sample such as the blood to be tested, and another liquid inlet, b, is connected to the sac of buffer containing detection beads. A small amount of antifoaming agent is pre-added to the buffer pool or a small amount of antifoaming agent is pre-applied to mixing reservoir 203.

The buffer solution along with some air is drawn into pump 400; then, the blood sample is also drawn into pump 400 from the inlet. During this process, the volume of the blood sample is detected and calculated by the fluid sensor at the valve. The two liquids in pump 400 are pushed into the mixing pool at the same time, and the remaining air in the pump pipe is also pushed into mixing reservoir 203, generating bubbles that help with the liquid mixing process. After the mixing is completed and the air bubbles are eliminated and the air rises to the top of mixing reservoir 203, the mixed liquid is then drawn back to pump 400, and is fed to the output end and into the detection area.

Although many air bubbles are being introduced when the buffer solution is drawn in, the mixed liquid has no air bubbles when it enters the detection area from the output end due to the presence of the antifoaming agent and the resting process in mixing reservoir 203. By using a liquid level sensor to control the valves and pump 400, it can effectively control a sufficient, but not excessive, amount of air to enter pump 400, and also prevent pump 400 from pushing excess air in mixing reservoir 203 out to the liquid channel at the output.

The above embodiments are only applicable for describing the present disclosure, but not for limiting the present disclosure. General technical personnel in the relevant technical field, without deviating from the spirit and scope of the present disclosure, can make various changes and modifications. Therefore, all equivalent technical solutions shall also fall within the scope of the present disclosure. The scope of patent protection of the present disclosure shall be defined by the claims.

## Claims

1. A microfluidic system suitable for liquid mixing, wherein the microfluidic system is comprised of the following:
at least two fluid inlets (101, 102), wherein the fluid inlets (101, 102) are respectively connected to the microfluidic channels (301, 302),
at least one pump (400), wherein the pump (400) is connected to the microfluidic channels (301, 302) to apply positive pressure or negative pressure to the microfluidic channels (301, 302) so that the sample or liquid can be transferred through the microfluidic channels (301, 302), and at least one pump (400) is also connected to the fluid outlets through the microfluidic channels (303, 304),
at least one valve (501, 502, 503, 504) set up in one or more of the microfluidic channels (301, 302, 303, 304) to selectively control the opening and closing of the microfluidic channels (301, 302, 303, 304), and
at least one mixing reservoir (203),
wherein the mixing reservoir (203) is connected to the pump (400) through the microfluidic channel (303).

2. A microfluidic system according to Claim 1, wherein the microfluidic channels (301, 302, 303, 304) are also comprised of at least one sensor (601, 602, 603, 604), the sensors (601, 602, 603, 604) detect the fluid flow in the microfluidic channels (301, 302, 303, 304) in order to control the action of the pump (400) and the valves (501, 502, 503, 504) when the volume of the liquid is drawn in by the pump (400).

3. A microfluidic system according to Claim 1 or 2, **characterised in that** it also includes at least two sample containers: the first sample container (201) and the second sample container (202), wherein the first sample container (201) and the second sample container (202) are used to hold different liquids; the first sample container (201) and the second sample container (202) are connected to the microfluidic channels (301, 302) through the two fluid inlets (101,102), and the liquid in the two sample containers (201, 202) can be transferred through the microfluidic channels (301, 302) under the action of the pump (400).

4. A microfluidic system according to Claim 1 or 2, **characterised in that** the sensors (601, 602, 603, 604) are liquid level sensors.

5. A microfluidic system according to Claim 1 or 2, **characterised in that** the pump (400) is a syringe pump.

6. A microfluidic system according to Claim 1 or 2, **characterised in that** the valves (501, 502, 503, 504) are pneumatically actuated membrane valves.

7. A microfluidic system according to Claim 2, **characterised in that** it also includes a control system, wherein through the fluid signal fed back by the sensors (601, 602, 603, 604), the system outputs a control signal to control the corresponding pump (400) and valves, thereby controlling the operation of the microfluidic system.

8. A method for mixing liquid in a microfluidic system, **characterised in that** the driving source in the microfluidic system pushes the liquid samples to be mixed into the mixing reservoir (203) through the microfluidic channels (301, 302) from the respective fluid inlets (101, 102),
the liquid drawn in from at least one fluid inlet (101, 102) is mixed with a certain amount of air; when the driving source pushes the liquid into the mixing reservoir (203), the air generates air bubbles, flows through the mixed liquid and rises to the top of the mixing reservoir (203),
the driving source draws the mixed liquid in the mixing reservoir (203) out from the mixing reservoir (203) and transfers it to the next channel through the outlet,
the driving source is a pump (400); and furthermore, preferably, the pump (400) is a syringe pump.

9. A method for mixing liquid in a microfluidic system according to Claim 8, **characterised in that** a liquid level sensor is set up in the mixing reservoir (203), so that when the mixed liquid is drawn out from the mixing reservoir (203), either sensor location will enable the valve connected to the mixing reservoir (203) and the pump (400) to close before the air is drawn so that the volume of mixed liquid drawn can be calculated and controlled.

10. A method for mixing liquid in a microfluidic system according to Claim 8 or 9, **characterised in that** an antifoaming agent is added to the system so that no excess air bubbles in the mixed liquid will accumulate at the top of mixing reservoir (203); preferably, the antifoaming agent is pre-added to the mixing reservoir (203), the pump (400) or the liquid sample to be mixed.
